Europäisches Patentamt
European Patent Office
Office européen des brevets

(11) Veröffentlichungsnummer: **0 263 502**
**A2**

# (12) EUROPÄISCHE PATENTANMELDUNG

(21) Anmeldenummer: 87114616.3

(22) Anmeldetag: 07.10.87

(51) Int. Cl.⁴: C12P 21/02 , C07K 5/00 , C07F 9/38 , A61K 37/02 , C12P 9/00 , //(C12P21/02,C12R1:125)

(30) Priorität: 09.10.86 DE 3634437

(43) Veröffentlichungstag der Anmeldung: 13.04.88 Patentblatt 88/15

(84) Benannte Vertragsstaaten: AT BE CH DE ES FR GB GR IT LI LU NL SE

(71) Anmelder: CIBA-GEIGY AG
Klybeckstrasse 141
CH-4002 Basel(CH)

(72) Erfinder: Loeffler, Wolfgang, Prof. Dr.
Gellertstrasse 11a
CH-4052 Basel(CH)
Erfinder: Kugler, Martin, Dr.
August-Lämmle-Strasse 29
D-7317 Wendlingen(DE)
Erfinder: Jung, Günther, Prof. Dr.
Ob der Grafenhalde 5
D-7400 Tübingen(DE)
Erfinder: Kern, Armin, Dr.
Burgholzweg 87
D-7400 Tübingen(DE)
Erfinder: Rapp, Claudius
Jusinus-Kerner-Strasse 13
D-7400 Tübingen(DE)

(74) Vertreter: Zumstein, Fritz, Dr. et al
Bräuhausstrasse 4
D-8000 München 2(DE)

## (54) Oligopeptid-Antibiotika.

(57) Rhizocticine der Formel

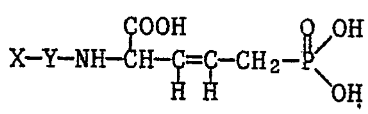

(I),

worin X Wasserstoff oder einen hydrophoben Aminosäurerest und Y einen basischen Aminosäurerest bedeuten, wobei die C2-Atome der Aminosäurereste die L-Konfiguration besitzen, sowie geschützte Derivate und Salze davon.

Die Verbindungen werden aus Kulturbrühen von Bacillus subtilis oder synthetisch gewonnen und haben insbesondere fungizide Wirkung.

EP 0 263 502 A2

Xerox Copy Centre

# Oligopeptid-Antibiotika

Die Erfindung betrifft neue Di-und Tripeptide, Verfahren zu ihrer Herstellung, Extrakte und Konzentrate aus Bakterienkulturbrühen enthaltend diese Oligopeptide in angereicherter Form, die Verwendung der neuen Peptide als Fungizide, Nematizide oder Herbizide, und pharmazeutische Präparate, Pflanzenschutzmittel und herbizide Mittel, die solche Peptide enthalten. Die Erfindung betrifft ferner ein Verfahren zur Herstellung eines Spaltproduktes aus diesen Peptiden in optisch reiner Form und dieses optisch reine Spaltprodukt selbst. Die neuen Di-und Tripeptide werden als Rhizocticine A, B, C, D etc. bezeichnet, das Spaltprodukt ist L-2-Amino-5-phosphono-3-cis-pentensäure (L-APPA). Rhizocticine haben antifungische, nematizide und herbizide Wirkungen.

Die Rhizocticine der vorliegenden Erfindung können aus Bakterien, insbesondere Bacillus subtilis, gewonnen werden. Antifungische Extrakte aus Bacillus subtilis, die als "Rhizoctonia factor" und "Aspergillus factor" bezeichnet werden, sind neben dem bekannten antibiotischen Polypeptid Subtilin [Jansen et al., Arch. Biochem. 4, 297 (1944)], durch H. David Michener et al., Arch. Biochem. 22, 208-214 (1949), bekannt geworden. Die beiden Faktoren unterscheiden sich in ihrem Wirkungsspektrum, indem der Rhizoctonia Faktor gegen Aspergillus niger, Aspergillus oryzae, oder Fusarium lycopersici im Gegensatz zum Apsergillus-Faktor praktisch keine Aktivität aufweist. Eine Reindarstellung der wirksamen Verbindungen dieser Faktoren und ihre Strukturaufklärung ist nicht bekannt geworden. Die vorliegenden reinen Rhizocticine sind gegen Rhizoctonia solani im Vergleich zum unreinen Rhizoctonia Faktor um zwei Zehnerpotenzen stärker wirksam.

Ebata et al., J. Antibiot. 22, 467-472 (1969) isolierten aus Bacillus subtilis PCI 219, der mit ATCC 6633 identisch ist, drei antifungische Metabolite der Bacillomycin-Gruppe, welche sie als Subsporine bezeichneten. Diese Antibiotika entsprechend dem Aspergillus-Faktor in wesentlichen biologischen und physikalischen Eigenschaften, so dass an der Identität nicht zu zweifeln ist.

Ebata et al. hatten den Rhizoctonia-Faktor und das zeitlich vorher erscheinende Bacilysin, ein gleich dem Rhizoctonia-Faktor niedermolekulares, hydrophiles Antibiotikum, übersehen.

Bacilysin, das Dipeptid N-Alanyl-3-(2,3-Epoxycyclohexanon-4-yl)-alanin (Walker et al., Biochem. J. 118, 563-565, 197), wurde erstmals 1946 (Forster et al., J. Bacteriol. 51, 363-369) ohne Angaben zur chemischen Struktur unter dem Namen Bacillin, 1949 als Bacilysin [Newton, Brit. J. Exptl. Pathol. 30, 306-319 (1949)] und 1973 nochmals als Tetain [Chmara et al., Biophys. Res. Commun. 52, 1382-1387 (1973)] beschrieben. In zwei Fällen waren die Produzenten Stämme der Art Bacillus subtilis und einmal Bacillus pumilus. Nach neueren Beobachtungen [Leoffler et al., J. Phytopathol. 15, 204-213 (1986)] wurde Bacilysin auch von Bacillus subtilis F-29-3, dem Fengymycin-Produzenten [Vanittanakom et al., J. Antibiot. 39, 888-901 (1986)] sowie von allen übrigen untersuchten Wildstämmen der Species Bacillus subtilis sowie von je einem Stamm der Arten Bacillus pumilus and Bacillus licheniformis in verschiedenen Medien gebildet. Die Substanz hemmt einige Bakterien, die Sprosspilze Saccharomyces cerevisiae und Candida albicans, ist aber im Gegensatz zum Rhizoctonia-Faktor gegen Hyphenpilze praktisch wirkungslos.

Eine gewisse Aehnlichkeit besteht zwischen Rhizocticin B und den Plumbemycinen A und B[Boo Kil. Park et al., Agr. Biol. Chem. 41, 573-579 (1977)], die jedoch aus Streptomyces (nicht enger verwandt mit dem Rhizocticin-Produzenten Bacillus) isoliert wurden. Die Uebereinstimmung betrifft auch nur den L-APPA-rest des Tripeptids Rhizocticin B, wohingegen die der L-APPA benachbarten Aminosäuren verschieden sind. Die Plumbenycine A und B haben gemäss Park et al. die Formeln L-Ala-L-Asp-D-APPA (A) und L-Ala-L-Asn-D-APPA (B), wobei die Zuordnung D zur APPA falsch sein dürfte. Der augenfälligste Unterschied besteht in den sich nicht überschneidenden, sehr verschiedenen Wirkungsspektren der Rhizocticine einerseits und der Plumbemycine andererseits. Plumbemycine wirken nur gegen Bakterien, Rhizoticine gegen Pilze.

Die Erfindung basiert auf der Isolierung, Reindarstellung und Strukturaufklärung der Rhizocticine A, B, C und D aus Kulturbrühen von Bakterien. Ausgehend von diesen näturlich vorkommenden Rhizoctricinen können analoge Verbindungen mit ähnlichen oder verbesserten Eigenschaften auf an sich bekannte Weise hergestellt werden.

Die Erfindung betrifft Rhizocticine der Formel

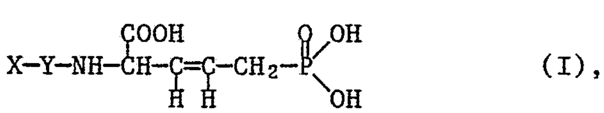

(I),

worin X Wasserstoff oder einen hydrophoben Aminosäurerest und Y einen basischen Aminosäurerest bedeuten, wobei die C2-Atome der Aminosäurereste die L-Konfiguration besitzen, sowie geschützte Derivate und Salze davon.

Die verwendeten Abkürzungen entsprechen den international anerkannten Nomenklaturregeln. In den abgekührzten Notierungen der Peptide ist die Aminogruppe auf der linken und die Carboxygruppen auf der rechten Seite zu denken.

Hydrophobe Aminosäurereste X sind von solchen Aminosäuren abgeleitet, die am C2-Atom einen hydrophoben Rest tragen und sind insbesondere L-Val, L-Ile, L-Leu oder L-Ala, X ist bevorzugt Wasserstoff oder L-Val.

Basische Aminosäurereste Y sind von solchen Aminosäuren abgeleitet, die am C2-Atom einen basischen Rest tragen und sind insbesondere L-Arg, L-Lys, L-Orn oder Homologe davon, z.B. L-HomoArg. Y ist bevorzugt L-Arg.

Die Aminosäure an der rechten Seite der Formel I hat die chemische Bezeichnung L-2-Amino-5-phosphono-3-cis-pentensäure und wird abgekürzt als L-APPA bezeichnet.

Bevorzugte Rhizocticine der Formel I sind diejenigen, worin X Wasserstoff und Y L-Arg (Rhizocticin A: L-Arg-L-APPA) oder X L-Val und Y L-Arg (Rhizocticin B: L-Val-L-Arg-L-APPA) bedeuten und ihre Salze.

Weiter bevorzugte Rhizocticine der Formel I sind solche worin X L-Ile und Y L-Arg (Rhizocticin C: L-Ile-L-Arg-L-APPA) oder X L-Leu und Y L-Arg (Rhizocticin D: L-Leu-L-Arg-L-APPA) bedeuten und ihre Salze.

Eine weitere bevorzugt Gruppe von Rhizocticinen der Formel I sind L-Val-L-Lys-L-APPA, L-Ala-L-Arg-L-APPA, L-HomoArg-L-APPA, L-Val-L-HomoArg-L-APPA, L-Ile-L-HomoArg-L-APPA, L-Leu-L-HomoArg-L-APPA, L-Ile-L-Lys-L-APPA, L-Leu-L-Lys-L-APPA, L-Ala-L-Lys-L-APPA, L-Val-L-Orn-L-APPA, L-Ile-L-Orn-L-APPA, L-Leu-L-Orn-L-APPA und L-Ala-L-Orn-L-APPA und ihre Salze.

Geschützte Derivate der Rhizocticine der Formel I sind solche, in denen eine oder mehrere funktionelle Gruppen in geschützter Form vorliegen. Solche Schutzgruppen sind in der Peptidchemie bekannt und können bei der chemischen Peptidsynthese zum Schutz der funktionellen Gruppen vor unerwünschter Reaktion benutzt werden, wie weiter unten ausgeführt ist. Eine besondere Art sind unter physiologischen Bedingungen abspaltbare Schutzgruppen, die auch nach der Synthese eingeführt werden können und die unter physiologischen Bedingungen, d.h. in vivo, abgespalten werden können.

Salze sind in erster Linie die inneren Salze der Rhizocticine der Formel I, aber auch Säureadditionssalze oder Salze mit Basen. Für pharmzeutische Zwecke hauptsächlichst physiologisch unbedenkliche Salze in Frage, die aber auch für Pflanzenschutzzwecke oder als Herbizide einsetzbar sind. Solche Salze sind Säureadditionssalze mit anorganischen Säuren, insbesondere Mineralsäuren, z.B. Salzsäure, Schwefelsäure oder Phosphorsäure, oder Salze mit organischen Carbon-, Sulfon-oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner Aminosäuren, sowie Methansulfonsäure, Ethansulfonsäure, 2-Hydroxyethansulfonsäure, Ethan-1,2-disulfonsäure, Benzolsulfonsäure, 4-Methyl-benzolsulfonsäure oder Naphthalin-2-sulfonsäure, oder mit anderen sauren organischen Verbindungen, wie Ascorbinsäure.

Rhizocticine mit freier Phosphono-und/oder Carboxylgruppen können Metall-oder Ammoniumsalze bilden, wie Alkalimetall-und Erdalkalimetall-, z.B. Natrium-, Kalium-, Magnesium-oder Calciumsalze, sowie Ammoniumsalze mit Ammoniak oder geeigneten organischen Aminen, wobei in erster Linie aliphatische, cycloaliphatische, cycloaliphatischaliphatische oder araliphatische primäre, sekundäre oder tertiäre Mono-, Di-oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxy-ethyl-diethyl-amino oder Tri-(2-hydroxyethyl)-amin, basische aliphatische Ester von Carbonsäuren, z.B. 4-Aminobenzoesuaure-2-diethylaminoethylester, Niederalkylenamine, z.B. 1-Ethylpiperidin, Cycloalkylamine, z.B. Dicyclohexylamin, oder Benzylamin, z.B. N,N'-Dibenzylethylendiamin, ferner Basen vom Pyridintyp, z.B. Pyridin, Collidin oder Chinolin.

Zur Isolierung oder Reinigung können auch pharmazeutisch oder phytologisch ungeeignete Salze Verwendung finden.

Die Erfindung betrifft auch die Verfahren zur Herstellung der Rhizocticine der Formel I, ihrer Derivate und Salze, dadurch gekennzeichnet, dass man

a) einen Rhizocticine produzierendern Bakterienstamm in einem Nährmedium, dass gegebenenfalls eine Aminosäure X oder Y oder ein Dipeptid X-Y enthält, züchtet und aus der Kulturbrühe das gewünschte Rhizocticin oder Rhizocticingemisch isoliert, oder

b) in beliebiger Reihenfolge die Aminosäuren X, Y und L-2-Amino-5-phosphono-3-cis-pentensäure, oder geschützte Derivate davon, peptidartig miteinander verknüpft, oder

c) zur Herstellung eines Rhizocticins der Formel I, worin X Wasserstoff ist, aus einem Rhizocticin der Formel I, worin X einen hydrophoben Aminosäurerest darstellt, X abspaltet. wenn notwendig, die Schutzgruppe(n) abspaltet, und eine erhaltenes Rhizocticin der Formel I in ein Salz oder ein erhaltenes Salz in die freie Verbindung überführt.

Verfahren a: Rhizocticine produzierende Bakterienstämme sind insbesondere Bacillus Arten, wie insbesondere Bacillus subtilis, z.B. ATCC 6633 oder F-29-3, oder gegebenenfalls auch Stämme der weiter vorn genannten Bacillus Arten, wie Bacillus pumilis oder Bacillus licheniformis.

Nährmedium für die Produktion von Rhizocticinen sind wässriger Natur und enthalten Kohlenstoff-und Stickstoffquellen, Mineralsalze und definierte oder komplexe organische Zusätze. Geeignete Nährmedien enthalten beispielsweise einen Zucker, wie Glucose oder Mannit, Glycerin, Glutaminsäure, Sojamehl, Hefeextrakt, Ammoniumsulfat, Natriumchlorid, Kaliumchlorid, Kaliumhydrogenphosphat, Calciumcarbonat, Magnsium-, Eisen-, Kupfer-und Mangansulfat, und dergleichen. Mit Vorteil wird anstelle von Glucose Mannit eingesetzt und zusätz lich eine Aminosäure, insbesondere Asparagin oder auch eine der für die Rhizocticinsynthese benötigten und in die Rhizocticine einzubauenden Aminosäuren oder Dipeptide.

Die Züchtung des Bakterienstammes erfolgt zwischen 15 und 33°C, bevorzugt bei 27°C, in üblichen Fermentern steigender Grösse unter aeroben Bedingungen, bevorzugt bis ein Maximum an Rhizocticinen gebildet ist.

Die Aufarbeitung der Kulturbrühe erfolgt nach an sich bekannten Methoden, z.B. nach Ansäuern auf pH $< 7$, bevorzugt etwa pH 2,5, mittels Salzsäure, durch Abtrennen der Bakterienzellen mittels Zentrifugation oder Filtration, Einengen der erhaltenen Bakterienfreien Kulturbrühe, z.B. durch Abdampfen, erneute Klärung z.B. durch Zentrifugation, Einengen zur Trockne, Aufnehmen in Aethanol, z.B. 70%-igem Aethanol, erneute Fällung durch Abkühlen, z.B. auf etwa 4°C, und erneutes Einengen des alkoholischen Ueberstandes. Das Einengen erfolgt bevorzugt durch Lyophilisation. Der erhaltene alkoholische Extrakt enthält die Rhizocticine in angereicherter Form.

Die weitere Reinigung erfolgt in saurer, wässriger Phase mit Adsorberharzen, z.B. Amberlite XAD-16, im Batchverfahren oder an einer Säule, und mittels Ausschluss-und Ionenaustauschchromatographie, z.B. an Sephadex CM-15 und Sephadex G-25, wobei als mobile Phasen deionisiertes Wasser, 5%-iger wässriger Ethylalkohol und Ammoniumacetat-Puffer pH 4 bis pH 5,5 dienen können. Durch wiederholte Ausschlusschromatographie, z.B. an Sephadex G-10 mit 15 % EtOH/85 % $H_2O$ als Laufmittel können die Rhizocticine A und B in reiner Form gewonnen werden. Eine weitere Reinigungsstufe stellt die Hochdruckflüssigkeitschromatographie dar, z.B. an RP18 Nucleosil.

Aus den vorgereinigten Rhizocticin B enthaltenden Fraktionen können die Rhizocticine C und D durch Hochdruckflüssigkeitschromatographie an RP18 Nucleosil gewonnen werden.

Zur Herstellung der anderen unter die Formel I fallenden Rhizocticine werden dem Nährmedium die entsprechenden anderen hydrophoben und basischen Aminosäuren H-X-OH bzw. H-Y-Oh, oder Dipeptide H-X-Y-OH zugesetzt und die Aufarbeitung auf analoge Weise durchgeführt.

Die Erfindung betrifft auch die aus den Kulturbrühen gewonnenen Extrakte und Konzentrate enthaltend die Rhizocticine in angereicherter Form.

Verfahren b: Die Verknüpfung von X, Y und L-APPA zum gewünschten Di-oder Tripeptid erfolgt durch Ausbildung einer Amidbindung zwischen zwei Fragmenten, nämlich der Carboxylgruppen des linken Fragmentes und der α-Aminogruppe des rechten Fragmentes. Fall X ebenfalls eine Aminonsäure ist, kann die Verknüpfung in verschiedener Reihenfolge geschehen indem beispielsweise zuerst das Fragment Y mit dem Fragment L-APPA und das Reaktionsprodukt mit X, oder zuerst X mit Y und das entstehende Dipeptidfragment X-Y mit L-APPA amidartig verknüpft wird.

Die zu benutzenden Methoden sind in der Peptidchemie allgemein bekannt.

Vorzugsweise wird die Reaktion so durchgeführt, dass man ein reaktionsfähiges Carbonsäurederivat des einen Fragments mit dem komplementären Fragment, das eine freie α-Aminogruppe aufweist, umsetzt, wobei die Aktivierung der Carboxylgruppe des Carbonsäurederivate, auch in situ erfolgen kann.

Reaktionsfähige Carbonsäurederivate sind in erster Linie reaktionsfähig aktivierte Ester oder reaktionsfähige Anhydride, ferner reaktionsfähige cyclische Amide; dabei können reaktionsfähige Säurederivate auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methodes des aktivierten Vinylesters), Carbamoylvinylester (die man z.B. durch Behandeln der entsprechenden Säure mit einem Isoxazoliumreagens erhalten kann; 1,2-Oxazolium-oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B.

durch Behandeln der entsprechenden Säure mit einem geeigneten N,N'-disubstituierten Carbodimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignete substiutierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlorphenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenylthioester (die man z.B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester), Amino-oder Amidoester (die man z.B. durch Behandeln der entsprechenden Säure mit einer N-Hydroxyamino-bzw. N-Hydroxy-amido-Verbindung, z.B. N-Hydroxy-succinamid, N-Hydroxy-piperidin, N-Hydroxy-phthalimid oder 1-Hydroxy-benztriazol, z.B. nach der Anhydrid-oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht ober Aminogruppen reagieren).

Säureanhydride können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydrochinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäure (die man z.B. durch Behandeln der entsprechenden Säure mit einem gegebenenfalls substituierten Niederalkan-oder Phenylalkancarbonsäurehalogenid, z.B. Phenylessigsäure-, Pivalinsäure-oder Trifluoressigsäurechlorid erhalten kann; Methode der gemischten Carbonsäureanhydride) oder mit organischen Sulfonsäure (die man z.B. durch Behandeln eines Salzes, wie eines Alkalimetallsalzes, der entsprechenden Säure, mit einem geeigneten organischen Sulfonsäurehalogenid, wie Niederalkan-oder Aryl-, z.B. Methan-oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethylpyrazol (die man z.B. über das Säurehydrazid durch Behandeln mit Acetylaceton erhalten kann; Pyrazolid-Methode).

Wie erwähnt, können die Carbonsäurederivate auch in situ gebildet werden. So kann man z.B. N,N'-disubstituierte Amidinoester in situ bilden, indem man das Gemisch des komplementären Fragments mit der freien Aminogruppe und des Peptidfragments mit freier Carboxylgruppe in Gegenwart eines geeigneten N,N-disubstituierten Carbodiimids, z.B. N,N'-Dicylcohexylcarbodiimid, zur Reaktion bringt. Ferner kann man Amino-oder Amidoester der Säure in Gegenwart des zu acylierenden Amins bilden, indem man das Gemisch der entsprechenden Säure-und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B .4-Dimethylamino-pyridin, umsetzt.

Alternativ kann Verfahrensvariante b) auch so durchgeführt werden, dass man ein Fragment mit freier Carboxylgruppe mit dem komplementären Fragment umsetzt, in dem die Aminosäure in reaktionsfähiger Form vorliegt. Die Aminogruppe kann z.B. durch Umsetzung mit einem Phosphit, z.B. Diethylchlorphosphit, 1,1-Phenylen-chlorphosphit, Ethyl-dichlor-phosphit, Ethylen-chlor-phosphit oder Tetraethylpyrophosphit, aktiviert werden.

Die Aminogruppe kann auch durch Bindung an Halogencarbonyl, z.B. Chlorcarbonyl, oder in Form einer Isocyanatgruppe aktiviert sein.

Freie funktionelle Gruppen in den Fragmenten, die, sofern sie nicht an der Reaktion teilnehmen sollen, darum vorteilhafterweise in geschützter Form vorliegen, sind Carboxyl-und Aminogruppen, sowie die Hydroxygruppen der L-APPA, die als saure Hydroxygruppen analog den Carboxylgruppen geschützt werden können.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Carboxylgruppen sind üblicherweise in veresterter Form geschützt, wobei solche Estergruppenierungen unter schonenden Bedingungen leicht spaltbar sind. In dieser Art geschützte Carboxylgruppen enthalten als veresternde Gruppen in erster Linie in 1-Stellung verzweigte oder in 1-oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butylcarbonyl, Arylmethoxycarbonyl mit einem oder zwei Arylresten, wobei diese gegebenenfalls, z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono-oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitroenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl-1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl-oder Stannylreste enthalten vorzugsweise Niederalkyl, insbesondere Methyl, als Substituenten der Silizium-oder Zinn-Atome. Entsprechende Silyl-oder Stannylgruppen sind in erster Linie Triniederalkylsilyl, insbesondere Trimethylsilyl, ferner Dimethyl-tert.-butyl-silyl, oder entsprechende substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acyl-niederalk-1-en-yl-amino-, Silyl-oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls,z.B. durch Halogen oder Aryl, substituierten Alkancarbonsäure oder gegebenenfalls, z.B. durch Halogen, Niederalkoxy oder Nitro, substituierten Benzoesäure, oder eine Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor-oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1-oder 2-Stellung geeignete substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro,mono-oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, oder 9-Fluorphenylmethoxycarbonyl, Aroylmethoxycarbonyl, worin die Aroylgruppe vorzugsweise gegebenenfalls, z.B. durch Halogen, wie Brom, substituiertes Benzoyl dargestellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unäbhängig voneinander je einen gegebenenfalls substituierten, z.B. durch Niederalkyl, Niederalkoxy, Aryl, Halogen, oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilyethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon-oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di-oder insbesondere Triarylmethylaminogruppe darstellt, sind die Arylreste insbesondere gegebenenfalls substituierte Phenylreste. Solche Gruppen sind beispielsweise Benzyl-, Diphenylmethyl-und insbesondere Tritylamino.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbestes. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor-oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl (BOC), z.B. 4-Nitrobenzyloxycarbonyl, oder Diphenylmethoxycarbonyl, oder 9-Fluorenylmethoxycarbonyl (Fmoc), oder 2-Halogen-niederalkoxycarbonyl, wie 2,2,2-Trichlorethoxycarbonyl, ferner Trityl oder Formyl.

Als Schutzgruppe, wie insbesondere Carboxylschutzgruppe, im Sinne dieser Anmeldung ist auch ein in leicht abspaltbarer Weise mit der zu schützenden funktionellen Gruppe, wie insbesondere Carboxylgruppe, verbundener polymerer Träger zu verstehen, wie er z.B. für die Merrifield-Synthese geeignet ist. Ein solcher geeigneter polymerer Träger ist z.B. ein durch Copolymerisation mit Divinylbenzol schwach vernetztes Polystyrolharz, das zur reversiblen Bindung der Peptidreste geeignete Brückenglieder trägt.

Die Reaktion kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs-oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittels sein kann, unter Kühlen oder Erwärmen, z.B. in einem Temperaturbereich von etwa -30°C bis etwa +150°C, in einem geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl-, N,N'-Dicyclohexyl-oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium-oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Verfahren c: Zur Abspaltung des hydrophoben Aminosäureester X aus einem Rhizocticin der Formel X-Y-L-APPA können übliche Amidspaltungsmethoden eingesetzt werden. Die Spaltung kann in saurem Medium, z.B. mit Salzsäure, durchgeführt werden, wobei durch entsprechende Einstellung des pH-Wertes, der Temperatur und Zeit eine weitergehende Hydrolyse des gewünschten Spaltprodukten der Formel Y-L-APPA vermieden wird.

Bevorzugt wird X enzymatisch mittels einer Protease, z.B. Thermolysin oder insbesondere Pronase, z.B. aus Streptomyces griseus, abgespalten.

Die Abspaltung der Schutzgruppen erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalystors, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetraniederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung eine 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbon säure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen-oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig-oder Trifluoressigsäure, gegebenenfalls in Gegenwart von Wasser gespalten, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine 9-Fluorenylmethoxycarbonylgruppe kann durch eine Base, insbesonders Piperidin oder Piperazin abgespalten werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die kata lytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Wenn erwünscht, werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator.

Die Herstellung von Salzen der Rhizocticine erfolgt nach den üblichen Methoden, z.B. durch Umsatz mit der entsprechenden Säure oder Base in einem geeigneten Lösungsmittel, wie Wasser oder Ethanol, und Abdampfen des Lösungsmittel, oder in einem unpolaren Lösungsmittel, wie Ether oder Tetrahydrofuran und/oder Pentan, und Ausfällen des Salzes. Zur Herstellung der Salze und der freien Verbindungen, die als innere Salze vorliegen, können auch Ionenaustauschharze auf an sich bekannte Weise eingesetzt werden.

Die Rhizocticine der vorliegenden Erfindung haben fungizide, nematizide und herbizide Wirkungen, wie sich in den folgenden Tests zeigen lässt. Für die Versuche zur biologischen Charakterisierung wurde ein Rhizocticin-Präparat mit einer spezifischen Aktivität von etwa 35 % verwendet.

Wirkung gegen Nematoden: Im Test gegen Caenorhabditis elegans wirken die Rhizocticine in Konzentrationen bis 10 µg/ml innerhalb von zwei Tagen letal gegen alle Nematoden. Bei Rhizocticin-Konzentrationen von 3,5 µg/ml ist die Bewegung der Nematoden gegenüber der Kontrolle reduziert.

Herbizide Wirkung: Bei 70 µg/ml ist das Wurzelwachstum von Lepidium sativum geringfügig reduziert.

Spektrum der durch Rhizocticine gehemmten Mikroorganismen:

-Wirkprofile: Pilze (Tabelle 1); Prokaryonten (Tabelle 2)

Die Hemmhofdurchmesser wurden jeweils für alle angegebenen Konzentrationen ermittelt. In den Zeilen sind von links nach rechts die wirksamen Konzentrationen angegeben (jeweils als mm Hemmhofdurchmesser), und wo sich rechts keine Zahlen mehr anschliessen, bedeuten die leeren Stellen "keine Wirkung", Ausnahme: Microsporum gypseum (unter "Onygenales ...") - dort war der Diffusionstest nicht auswertbar (allerdings zeigte sich Aktivität bei Anwendung eines anderen Testverfahrens).

MIC, IC50-und IC10-Werte für Fadenpilze wurden wie folgt besimmt: Rhizocticin-Lösungen in 35 % Ethanol wurden in verschiedenen Konzentrationen hergestellt. 50 µl Lösungen wurden zu 5 ml sterilem, 45°C warmem Malzagar hinzugegeben und in sterile Petrischalen (55 mm Durchmesser) gegossen. Die Konzentrationen an Rhizocticin waren 3,5, 1,1, 0,35, 0,11, 0,035 und 0,011 µg/ml. Die Platten wurden mit zylindrischen Agarstückchen (6 mm Durchmesser) aus der Randzone wachsender Pilzkolonien zentral beimpft. Während eines Zeitraumes von zwei bis zehn Tagen wurden der Durchmesser der wachsenden Kolonien gemessen. Die erhaltenen Messdaten wurden zur Bestimmung der minimalen Hemmkonzentrationen (MIC) sowie der Konzentrationen, bei denen das Antibiotikum eine 50%-ige (IC 50) bzw. eine 10%-ige Wachstumshemmung (IC 50) verursacht, herangezogen, Kontrollplatten ohne Antibiotikum dienten als Referenz (100 % Wachstum).

Die minimalen Hemmkonzentrationen für Sprosspilze (MIC) wurden in Mikrotiterplatten (24 Vertiefungen, 16 mm Durchmesser; Costar, Cambridge, USA) besimmt. Diese enthalten pro Vertiefung 940 µl Malzmedium, 50 µl einer über Nacht in Malzmedium herangezogenen Vorkultur sowie 10 µl einer Rhizocticin-Lösung in 35%-igem Ethanol.

Die Rhizocticin-Endkonzentrationen lagen im Bereich von 3,5 bis 0,011 µl/ml. Nach 24 Stunden Inkubationsdauer wurden diejenigen Konzentrationen, in der Verdünnungsreihe als MIC bezeichnet, bei denen gerade kein Wachstum der Keime festgestellt werden konnte.

In der Tabelle bedeuten Zeilen ohne Zahlenangaben in den entsprechenden Spalten, dass keine Tests (MIC, IC 50, IC 10) ausgeführt worden sind.

Tabelle 1: Antifungische Wirkung des Rhizocticins gegen Pilze (Malzmedium).

Hemmhoftyp:
rr sehr schwach bewachsen
W schwach bis mittelstark bewachsen
W+ stark bewachsen
* Test an der Riesenkolonie

| PILZE | Hemmhofdurchmesser (mm) bei pro Rondelle aufgetragener Menge (ng) | | | | | | Hemmhof-typ | MIC | IC 50 (µg/ml) | IC 10 |
|---|---|---|---|---|---|---|---|---|---|---|
| | $3{,}5 \cdot 10^3$ | $1{,}1 \cdot 10^3$ | $3{,}5 \cdot 10^2$ | $1{,}1 \cdot 10^2$ | $3{,}5 \cdot 10^1$ | $1{,}1 \cdot 10^1$ | | | | |
| ZYGOMYCOTA | | | | | | | | | | |
| Basidiobolus microsporus | 48 | 39 | 34 | | | | n | | | |
| Entomophthora coronata | 35 | | | | | | W+ | | | |
| Mycotypha africana | 22 | 17,5 | 13 | 10 | | | W | | | |
| Rhizomucor miehei | 32 | 27 | 23 | 21 | | | W | >3,5 | >3,5 | 0,11 |
| ASCOMYCOTA UND DEUTEROMYCOTA | | | | | | | | | | |
| Endomycetes und zugeordnete Blastomycetes | | | | | | | | | | |
| Candida albicans | 21 | 19 | 13 | | | | W | ~3,5 | | |
| Candida guilliermondii | 19 | 14 | + | | | | n | >3,5 | | |
| Dipodascus magnusii | 32 | 26 | 14 | | | | n | >3,5 | | |
| Nematospora coryli | 49 | 40 | 31 | | | | W+ | >3,5 | | |
| Saccharomyces cerevisiea | 25 | 21,5 | 17 | 12,5 | | | n | ~0,35 | | |
| Saccharomycopsis lipolytica | 37 | 33,5 | 28 | 23 | 17 | + | n | 0,35 | | |
| Schizosaccharomyces pombe | 54 | 48 | 23,5 | 23,5 | + | | n | 0,35 | | |
| Eurotiales und zugeordnete Deuteromycota | | | | | | | | | | |
| Aspergillus fumigatus | 29 | 24 | 19 | + | | | W | | | |
| Aspergillus terreus | 20 | | | | | | W | | | |
| Paecilomyces variotii | 46 | 41 | 34 | 31 | 20 | 14 | n | >3,5 | 1,1 | 0,11 |
| Penicillium notatum | 36 | 26 | 20 | + | | | W | | | |

| PILZE | Hemmhofdurchmesser (mm) bei pro Rondelle aufgetragener Menge (ng) $3{,}5 \cdot 10^3$ | $1{,}1 \cdot 10^3$ | $3{,}5 \cdot 10^2$ | $1{,}1 \cdot 10^2$ | $3{,}5 \cdot 10^1$ | $1{,}1 \cdot 10^1$ | Hemmhof-typ | MIC | IC 50 (µg/ml) | IC 10 |
|---|---|---|---|---|---|---|---|---|---|---|
| Onygenales und zugeordnete Deuteromycota | | | | | | | | | | |
| Epidermomyces floccosus | + | | | | | | W+ | | | |
| Microsporum gypseum | | | | | | | | >3,5 | >3,5 | 0,11 |
| Trichophyton erinacei | 44 | 37 | 23 | | | | W | >3,5 | >3,5 | 0,11 |
| Trichophyton mentagrophytes | 39 | 27 | 17 | | | | W+ | | | |
| Trichophyton rubrum | 44 | 28 | 15 | | | | W | >3,5 | >3,5 | 3,5 |
| Sphaeriales und zugeordnete Deuteromycota | | | | | | | | | | |
| Fusarium sp. Tü 8014 | 17,5 | 14 | 11 | | | | n | | | |
| Sordaria macrospora | 35 | 20 | + | | | | n | | | |
| Pezizales | | | | | | | | | | |
| Ascobolus carbonarius | 28 | | | | | | * | | | |
| Ascodesmis sphaerospora | 52 | 47 | 39 | 33 | 19 | | n | 3,5 | 0,35 | 0,035 |
| Plicaria anthracina | 37 | 25 | 25 | | | | * | 3,5 | 0,35 | 0,035 |
| Dothideales und zugeordnete Deuteromycota | | | | | | | | | | |
| Alternaria kikuchiana | 38 | 30 | 19 | + | | | W | | | |
| Cladosporium carrionii Nr. 1 | 17 | | | | | | W+ | | | |
| Cladosporium trichoides Nr. 3,4 | - | | | | | | | | | |
| Curvularia lunata | 28 | 18 | 14 | | | | W | | | |
| Stemphylium sarciniforme | 44 | 37 | 29 | 20 | | | W | | | |
| Stemphylium sp. Tü 609 | 42 | 34 | 27 | 24 | | | n | | | |

| PILZE | Hemmhofdurchmesser (mm) bei pro Rondelle aufgetragener Menge (ng) $3{,}5 \cdot 10^3$ | $1{,}1 \cdot 10^3$ | $3{,}5 \cdot 10^2$ | $1{,}1 \cdot 10^2$ | $3{,}5 \cdot 10^1$ | $1{,}1 \cdot 10^1$ | Hemmhof-typ | MIC | IC 50 (µg/ml) | IC 10 |
|---|---|---|---|---|---|---|---|---|---|---|
| Dematiaceae (Schwärzepilze) ungewisser Zuordnung | | | | | | | | | | |
| Exophiala mansonii Nr. 5,6,10 | - | | | | | | W+ | | | |
| Exophiala werneckii | 16 | | | | | | W+ | | | |
| Rhinocladiella compacta | 16 | | | | | | W+ | | | |
| Rhinocladiella pedrosoi | 16 | | | | | | W+ | | | |
| BASIDIOMYCOTA UND ZUGEORDNETE DEUTEROMYCOTA | | | | | | | | | | |
| Rhizoctonia solani | - | | | | | | * | ~3,5 | 0,35 | 0,11 |
| Rhodotorula rubra | + | | | | | | W+ | >3,5 | | |
| Ustilago maydis | - | | | | | | | >3,5 | | |

Anhang zu Tabelle 1:

IC 10 entspricht 10%-ige Wachstumshemmung

IC 50 entspricht 50%-ige Wachstumshemmung

MIC Minimale Hemmkonzentration

Tabelle 2: Rhizocticin-unempfindliche Prokaryonten (keine Hemmung bei Prüfung gegen 3,5 µg Rhizocticin pro Rondelle).

| | Medium, Temperatur (°C) |
|---|---|
| GRAM-POSITIVE BAKTERIEN | |
| Arthrobacter aurescens | NB, 27 |
| Arthrobacter oxydans | NB, 27 |
| Bacillus cereus | nHA, 37 |
| Bacillus subtilis | Ox, MB, 37 |
| Brevibacterium flavum | Ox, 37 |
| Clostridium pasteurianum | Clost, 27 |
| Corynebacterium insidiosum | Ox, 27 |
| Micrococcus luteus | NB, 27 |
| Staphylococcus aureus | Ox, 37 |
| Streptomyces glaucescens | nHA, 27 |
| Streptomyces violaceoruber | nHA, 27 |
| Streptomyces viridochromogenes | nHA, 27 |
| GRAM-NEGATIVE BAKTERIEN | |
| Achromobacter geminiani | nHA, 37 |
| Escherichia coli | Ox, MB, 37 |
| Proteus mirabilis | NB, 37 |
| Proteus vulgaris | NB, 37 |
| Pseudomonas fluorescens | NB, 27 |
| Salmonella typhimurium | nHA, 37 |

Testmedien: Die Angaben für Testmedien beziehen sich auf 1 ℓ entionisiertes Wasser
NB-Medium: Nutrient Broth 8,0 g, pH 7,0
nHA-Medium: Hefeextrakt 4,0 g, Malzextrakt 10,0 g, Glucose 4,0 g, pH 7,3
Oxo-Medium: Ox Lab Lemco Fleischextrakt 10,0 g, Pepton aus Casein 10,0 g, Nacl 5,0 g, pH 7,2
MB-Medium: Glucose 8,0 g, NaCl 5,0 g, Di-Ammoniumtartrat 4,0 g, $MgSO_4$ x $7H_2O$ 1,0 g, $K_2HPO_4$ 2,0 g, $CaCl_2$ 200 mg, $MnSO_2$ x $H_2O$ 10 mg, Ferrioxamin B 20,0 mg, pH 7,0
Clost-Medium: Pepton aus Casein 20,0 g, Glucose 50,0 g, Malzextrakt 3,0 g, Ox Lab Lemco Fleischextrakt 3,0 g, Hefeextrakt 30, g, Ascorbinsäure 200,0 mg, pH 7,0
MA-Medium: Malzextrakt 20,0 g, pH gemessen 6,0
Für feste Nährmedien wurden 20 gl Difco-Agar hinzugefügt.

Die Erfindung betrifft auch die Verwendung der Rhizocticine der Formel I und ihrer Salze und der Rhizocticine enthaltenden Extrakte oder Konzentrate als Fungizide, Nematizide oder Herbizide.

Die Erfindung betrifft auch pharmazeutische Präparate, die als Wirkstoff eine wirksame Dosis eines der erfindungsgemässen Rhizocticine oder ein Salz davon zusammen mit einer signifikanten Menge eines pharmazeutischen Trägermaterials enthalten, insbesondere solche intravenösen, oder topischen Applikation an Warmblüter, wie in allererester Linie den Menschen.

Die Dosierung des Wirkstoffes hängt von der Warmblüter-Spezies, dem Körpergewicht, Alter und dem individuellen Zustand, der zu behandelnden Krankheit sowie von der Applikationsweise ab. Die Dosis pro Warmblüter von etwa 70 kg Gewicht beträgt im allgemeinen etwa 50 bis 500 mg.Die erfindungsgemässen pharmazeutischen Präparaten eignen sich zur oralen oder parenteralen, z.B. i.v., i.m. oder topischen Verabreichung.

Die erfindungsgemässen pharmazeutischen Präparaten eignen sich zur oralen oder parenteralen, z.B. i.v., i.m. oder insbesondere zur topischen Verabreichung.

Beispielsweise verwendet man die Wirkstoffe der Formel I der vorliegenden Erfindung in Form von injizierbaren, z.B. intravenös, verabreichbaren Präparaten oder Infusionslösungen. Solche Lösungen sind vorzugsweise isotopische wässrige Lösungen oder Suspensionen, welche z.B. aus lyophilisierten Präparaten, welche den reinen Wirkstoff oder den Wirkstoff zusammen mit einem Trägermaterial, z.B. Mannit, enthalten, vor Gebraucht hergestellt werden können. Pharmazeutische Präparate zur oralen Anwendung sind gegebenenfalls sterilisiert und können Hilfsstoffe, z.B. Konservier-, Stabilisier-, Netz-und/oder Emulgiermittel, Löslichkeitsvermittler, Salze zur Regulierung des osmotischen Druckes und/oder Puffer enthalten. Für die topische Anwendung eignen sich insbesondere Sprays, Lotions und Pulver, auch Zäpfchen, z.B.Vaginalzäpfchen, die auf übliche Weise hergestellt werden. Die vorliegenden pharmazeutischen Präparate, die, wenn erwünscht, weitere pharmakologisch wertvolle Stoffe, z.B. andere Wirkstoffe, enthalten können, enthalten etwa 0,1 % bis 100 %, insbesondere etwa 1 % bis zu 100 %, des Wirkstoffes.

Die pharamazeutischen Präparate werden in an sich bekannter Weise, z.B. mittels konventioneller, in Pharmakopöen beschriebener Verfahren, hergestellt.

Die pharmazeutischen Präparate der vorliegenden Erfindung können in einem Verfahren zur therapeutischen Behandlung des menschlichen oder tierischen Körpers angewendet werden, z.B. zur Behandlung von Infektionen, welche durch Pilze oder Nematoden verursacht werden.

Weitere Verwendungsmöglichkeiten für die Rhizocticine der Formel I und ihre Salze bestehen im Pflanzenschutz, z.B. bei der Bekämpfung von Pilzkrankheiten von Pflanzen, z.B. von Gewächshauskulturen, bei der Pflege von infizierten Bäumen etc. Für diese Verwendung eignen sich z.B. Aerosole. Dafür können auch rohe Extrakte und Konzentrate aus Rhizocticine produzierenden Bakterienstämmen eingesetzt werden.

Die Erfindung betrifft daher auch Pflanzenschutzmittel enthaltend ein Rhizoticin der Formel I oder ein pflanzenverträgliches Salz davon oder Rhizocticine enthaltende Extrakte oder Konzentrate aus Rhizocticine produzierenden Bakterienstämmen, insbesondere aus Bacillus subtilis ATCC 6633.

Die Pflanzenschutzmittel enthalten eine fungizid wirksame Menge an Rhizocticinen in pulverförmiger oder gelöster Form. Bevorzugt erfolgt die Anwendung in versprühbarer wässriger Lösung.

Die Erfindung betrifft ferner die Verwendung der Rhizocticine als herbizide Mittel. Solche herbiziden Mittel enthalten ein Rhizocticin der Formel I oder ein Salz davon, oder Rhizocticine enthaltende Extrakte oder Konzentrate aus Rhizocticine produzierenden Bakterienstämmen, insbesondere aus Bacillus subtilis ATCC 6633.

Die Erfindung betrifft ferner ein Verfahren zur Herstellung optisch reiner L-2-Amino-5-phosphono-3-cis-pentensäure (L-APPA), dadurch gekennzeichnet, dass man ein durch Fermentation eines Rhizocticine produzierenden Bakterienstämmes erhaltenes Rhizocticin oder Rhizocticingemisch hydrolytisch spaltet und die L-APPA in optisch reiner Form isoliert.

Die wie weiter vorn beschrieben hergestellten Rhizocticine, insbesondere Rhizocticin A, B, C oder D, oder Mischungen davon, werden auf an sich bekannte Weise hydrolytisch in die einzelnen Aminosäuren aufgespalten. Die hydrolytische Spaltung kann unter sauren Be. dingungen, z.B. mit 6N Salzsäure bei Temperaturen bis zu etwa 110°C, oder bevorzugt enzymatisch, z.B. durch Behandeln mit einer Protease, z.B. Thermolysin, Carboxypeptidase A oder Trypsin, bei Temperaturen zwischen Zimmertemperatur und 45°C, bevorzugt, je nach Enzym, bei ca. 29°C bis 41°C, durchgeführt werden.

Die Isolierung von L-APPA erfolgt chromatographisch, z.B. mittels Ionenaustauschern, wie Dowex 50 WX8, oder eine der in der Peptidchemie üblichen Reinigungsmethoden.

Die Erfindung betrifft auch die optisch reine L-2-Amino-5-phosphono-3-cis-pentensäure und Salze davon.

Salze der L-APPA sind diejenigen mit den gleichen Säuren und Basen die unter den Rhizocticin Salzen aufgeführt sind.

## Beispiele

In den Beispielen werden die folgenden Kulturmedien verwendet.

Komplexes Produktionsmedium (T-Medium):

Glucose 15,0 g
Glycerin 10,0 g
Sojamehl 15,0 g
$(NH_4)SO_4$ 5,0 g
Hefeextrakt 1,0 g
NaCl 5,0 g
$CaCO_3$ 5,0 g
entionisiertes Wasser 1 ℓ
pH 7,3

Definiertes Produktionsmedium (PL-Medium):

Mannit 20,0 g
Asparagin 1,0 g
Glutaminsäure 5,0 g
$KHPO_4$ 1,0 g
$MgSO_4 \times 7\ H_2O$ 500,0 mg
KCl 500,0 mg
$FeSO_3 \times 7\ H_2O$ 0,15 mg
$CuSo_3 \times 5\ H_2O$ 0,16 mg
$MnSO_4$ 5,0 mg
entionisiertes Wasser 1 ℓ
pH 6,0

Der in den Beispielen verwendete Mikroorganismus Bacillus subtilis ist bekannt und kommerziell erwerbbar, z.B. unter der Bezeichnung ATCC 6633 von der Americn Type Culture Collection.
Der gleiche Stamm ist auch von den folgenden Hinterlegungsstellen erhältlich (Hinterlegungsstelle/Bezeichnung): National Collection of Industrial Bacteria/NCIB 8054, Institute for Fermentation/IFO 13720, National Collection of Type Cultures/NCTC 10400, Institute of Applied Microbiology/IAM 1069, Deutsche Sammlung von Mikroorganismen/DSM 347.

Der verwendete Stamm ATCC 6633 wurde am 2. Februar 1987 gemäss dem Budapester Vertrag nochmals bei der Deutschen Sammlung von Mikroorganismen, Grisebachstr. 8, D-3400 Göttingen, hinterlegt und erhielt die Eingangsnummer DMS 3973 zugeteilt (Hinterlegt Bezugszeichen BS6633).

Die folgenden Abkürzungen werden benutzt:
AS Aminosäure
Boc tert.-Butyloxycarbonyl
DC Dünnschichtchromatographie
DCC Dicyclohexylcarbodiimid
DCH Dicyclohexylharnstoff
DMF Dimethylformamid
Fmoc 9-Fluorenylmethoxycarbonyl
GC Gaschromatographie
HOBt Hydroxybenotriazol
OSu N-Hydroxysuccinimidester
RT Raumtemperatur
TFE Trifluoressigsäure

Beispiel 1: Fermentation im Kolbenmassstab:

500 ml Erlenmeyerkolben mit einem seitlichen Einstich (Schikane), die 100 ml Nährlösung (Medium PL) enthalten, werden mit je 0,4 ml Sporensuspension von ATCC 6633 beimpft und auf einer rotierenden Schüttelmaschine bei 120 rpm und 27°C ca. 72 h inkubiert.

Beispiel 2: Fermentation im 20 ℓ Massstab:

Die Fermentation wird in einem Bioreaktor mit Instrumenteneinheit zur Messung und Regelung von Propellerdrehzahl, Temperatur, pH, Belüftung und Antischaummittelzugabe durchgeführt. Die Umwälzung der Nährlösung im Rührkessel wird durch ein Intensor-Umwurfsystem gewährleistet. Der Reaktor wird mit 19 ℓ Nährlösung (PL) gefüllt und in situ sterilisiert (20 Min. bei 121°C und 1 atm Ueberdruck). Nach dem Abkühlen wird mit 5 % einer mindestens 10 Tage alten, gut versporten Kultur (siehe Beispiel 1) beimpft und bei 27°C, 800 rpm und 5 ℓ/Min. Luftdurchsatz inkubiert. Zur Unterdrückung der Schaumbildung wird nach Bedarf Antischaummittel hinzudosiert.

Die Ernte erfolgt nach ca. 60 Stunden, wenn das Produktionsmaximum erreicht ist, durch das Abzentrifugieren der Zellmasse.
Figur 1 zeigt eine Fermentation im 20 ℓ-Massstab.

Beispiel 3: Fermentation im 200 ℓ Massstab

Die Produktion der gewünschten Verbindungen wird in einem Bioreaktor mit Instrumenteneinheit zur Messung und Regelung von Propellerdrehzahl, Temperatur, pH, Belüftung und Antischaummittelzugabe mittels Schaumsonde durchgeführt. Die Belüftung erfolgt submers in einem Intensor-Umwurfsystem. Die Sterilisation erfolgt in situ mit 200 ℓ Nährlösung (PL) nach einem internen Programm. Das Impfmaterial, 5 ℓ einer unter Beispiel 1 beschriebenen, mindestens 10 Tage alten Vorkultur wird direkt über ein Schlauchleitung eingepumpt. Fermentationsparameter: 27°C, 800 rpm (Propellendrehzahl) und 50 ℓ/Min. Luftdurchsatz.

Die Ernte erfolgt nach ca. 60-80 Stunden, wenn ein Maximum der Konzentration der gewünschten Verbindungen erreicht ist.

Nach der Ernte können die Verbindungen aus dem Kulturfiltrat mit den üblichen chemisch-pysikalischen, vorwiegend chromatographischen und spektroskopischen Methoden isoliert werden.

Ausbeute der Rhizocticine in Bezug auf das Kulturfiltrat:
ca. 30 mg/ℓ, d.h. 60 g pro Fermenter.
Figur 2 zeigt eine Fermentation im 200 ℓ Massstab.

Beispiel 4: Aufarbeitung aus PL-Medium im 5 ℓ Massstab mittels Amberlite XAD-16 (Batchverfahren) und Sephadex CM-25 und G-15

5 ℓ Kulturbrühe werden mit 5N HCl auf pH 2,5 eingestellt. Der entstandene Niederschlag wird in einer Sorvall-Zentrifuge mit einem GSC 3 Rotor bei 10 000 g (ca. 8 000 rpm) abgetrennt und verworfen. In der Folge wird der Ueberstand (4.5 ℓ) bei 50° zur Trockene eingeengt und in 500 ml 70 % Ethanol aufgenommen.

Bei 4°C wird eine erneute Fällung durchgeführt und das Präzipitat vom hockaktiven Ueberstand getrennt. Der Niederschlag wird zweimal mit je 200 ml 70 % Ethanol extrahiert und die aktiven Ueberstände werden vereinigt und eingeengt.

Anschliessend wird das bräunliche Konzentrat in 500 ml deionisiertem Wasser aufgenommen, auf pH 3 eingestellt und im Batchverfahren mit 150 g Amberlite XAD-16 eine Stunde lang gerührt. Hierdurch können melaninartige und lipophile Verunreinigungen an das Absorberharz gebunden werden, während die Rhizocticine nicht adsorbiert werden. Das Adsorberharz wird abgetrennt, mit 200 ml deionisiertem Wasser gewaschen und die wässrigen Fraktionen vereinigt. Der Reinigungsvorgang mit Amberlite XAD-16 wird wiederholt und das gesamte wässrige Volumen eingeengt.

Das Lyophilisat wird in 10-15 ml 5 % Ethanol aufgenommen und durch eine Gelfiltration an Sephadex G-15 mit 5 % Ethanol weiter gereinigt. Die aktiven Fraktionen werden vereinigt, lyophilisiert und in 2 ml 5 % Ethanol aufgenommen. Nachfolgend wird ein zweiter Chromatographiedurchgang mit einer weiteren G-15 Säule durchgeführt.

Die lyophilisierten, aktiven Fraktionen des vorhergehenden Schrittes werden in 2 ml 5 mM Ammoniumacetat-Puffer pH 4 gelöst und auf eine Sephadex CM-25 Säule aufgetragen. Während die meisten Verunreinigungen bei oben genanntem Puffer nicht an den schwachen Ionenaustauscher binden, werden die Rhizocticine zurückgehalten. Bei der Elution mit 100 mM Ammoniumacetat-Puffer pH 4 können die Rhizocticine A und B (frühere Bezeichnung Rhizocticine I bzw. III) voneinander getrennt werden.

Die Endreinigung der Rhizocticine erfolgt mittels Ausschlusschromatographie an Sephadex G1-5.

Beispiel 5: Aufarbeitung aus PL-Medium im 25 ℓ-und 200 ℓ-Massstab mittels einer Amberlite XAD-16 Säule und Sephadex CH-25 und C-15

Bei der Aufarbeitung von 25 bzw. 200 ℓ-Fermentern wird die Zellmasse auf pH 2,5 bis 3 eingestellt und mit einer Schlammzentrifuge abgetrennt. Ein sofortiges Einengen am Dünnschichtverdampfer oder mit ähnlichen Verfahren ist wegen stark schäumender Fermentationsprodukte nicht oder nur mit sehr grossen Schwierigkeiten mit PL-Medium als Basis durchführbar.

Durch Säulenchromatographie an Amberlite XAD-16 (1/10 des Ausgangs volumens) werden die störenden Detergentien sowie ein Teil der lipophilen und melaninartigen Verbindungen gebunden.

Der wässrige Durchlauf und das Waschwasser werden bei 60°C am Dünnschichtverdampfer eingeengt und lyophilisiert.

Die weitere Aufreinigung der Rhizocticine erfolgt in Anlehnung an Beispiel 4.

Beispiel 6: Aufarbeitung aus PL-Medium im 5 ℓ-Massstab mittels Bio-Gel P2, Sephadex CM-25 und Sephadex G-10

5 ℓ Kulturbrühe werden mit 5N HCl auf pH 2,5 eingestellt. Der entstandene Niederschlag wird in einer Sorvall-Zentrifuge mit einem GSG 3 Rotor bei 10 000 g (ca. 8 000 rpm) abgetrennt und verworfen. In der Folge wird der Ueberstand (4,5 ℓ) bei 30-40°C zur Trockene eingeengt und in 500 ml 70 % Ethanol aufgenommen.

Bei 4°C wird eine erneute Fällung durchgeführt und das Präzipitat vom aktiven Ueberstand getrennt. Der Niederschlag wird zweimal mit je 200 ml 70 % Ethanol extrahiert, die aktiven Ueberstände vereinigt und eingeengt.

Das aktive Lyophilisat (27,5 g) wird durch Gelfiltration an Bio-Gel P2 (200-400 mesh) mit reinem Wasser als Laufmittel weitergereinigt: v: 70 ml/h; Gelbett: 875 mm x 50 mm; 10 ml/Fraktion. Pro Biogel-Lauf werden hierzu 9,1 g (obige Gesamtmenge in 3 Aliquots aufgeteilt), gelöst in 10-15 ml deionisertem Wasser, aufgetragen. Die aktive Fraktionen (Fr. 98-124) werden zusammengefasst und lyophilisiert.

Zur Weiterreinigung wird das gesamte Lyophilisat (1,85 g in 2 ml 100 mM Ammoniumacetat gelöst) auf eine Sephadex CM-25-Säule, äquilibriert mit 100 mM Ammoniumacetat in Wasser (pH 5,5) aufgegeben. Die Elution erfolgt ebenfalls mit diesem Startpuffer. v: 50 ml/h; 700 mm x 30 mm Gelbett; 7 ml/Fraktion. Die aktiven Fraktionen werden vereinigt und dreimal zur weitgehenden Pufferentfernung lyophilisiert (230 mg Ausbeute).

Zur Auftrennung in Rhizocticin A und B und gleichzeitiger Feinreinigung wird eine Ausschlusschromatographie an Sephadex G-10 mit 15 % EtOH/85 % $H_2O$ als Laufmittel durchgeführt:
v: 16 ml/h; 4 ml/Fraktion; Gelbett: 950 mm x 20 mm
Die gesamte antifungische Aktivität wird in den Fraktionen 30-46 eluiert.
Dabei treten zwei Aktivitätsmaxima auf: Rhizocticin B in Fraktion 33
Rhizocticin A in Fraktion 41

Die Zuordnung erfolgt mittels DC-Analyse (System I, Ninhydrin-Detektion). Die reinen Fraktionen 30-34 (Rhiz.B) und 39-46 (Rhiz.A) werden jeweils vereinigt (Rhiz.B 13 mg und Rhiz.A 21 mg).

Mittels Hochdruckflüssigkeitschromatographie lässt sich nun die Einheitlichkeit von Rhizocticin A und B überprüfen und die Mischung 35-38 in die reinen Komponenten A und B auftrennen:

Reinheitskontrolle für Rhizocticin A und B

Säule: RP18 Nucleosil, 5µ; 4,6 x 250 mm, Vorsäule 4,6 x 10 mm
Mobile Phase: 0,1 % wässrige TFA (A)/ACN (B) 10 Min. isokratisch 100 % A, dann linearer Gradient von 0 % B auf 100 % B in 30 Min., Fluss: 1 ml/Min.

Semipräparative Trennung des Konzentrats der Mischfraktionen

Säule: RP18 Nucleosil, 5µ; 8 x 250 mm, Vorsäule 8 x 40 mm
Mobile Phase: 0,1 % wässrige TFA; Fluss 3 ml/Min.

Die Strukturerklärung von Rhizocticin A und B erfolgt nach üblichen Methoden, insbesondere durch Aminosäureanalyse des Totalhydrolysates, Aufklärung der zunächst unbekannten Aminosäure L-APPA mittels NMR $^{31}P$, $^{13}C$ und $^{1}H$ und Konfigurationsbestimmung durch gaschromatographischen Vergleich mit DL-5-Phosphonovaleriansäure mit dem hydrierten Hydrolysegemisch (TFA/Trimethylderivat) sowie Sequenzanalyse durch kombinierten Dansyl-Edman-Abbau (Dansyl-AS, PTH-AS/DC, GC), DNP-Derivate und C-Terminus-Bestimmung durch Reduktion mit $B_2H_6$ zu Aminoalkoholen.

Charakterisierung von Rhizocticin A, B und L-APPA

| | Rhiz. A | Rhiz. B | L-APPA |
|---|---|---|---|
| Natur | farbl. Pulver | farbl. Pulver | farbl. Pulver |
| Molekularformel | $C_{11}H_{22}N_5PO_6$ | $C_{16}H_{31}N_6PO_7$ | $C_5H_{10}NPO_5$ |
| Molmasse | 351 | 450 | 195 |
| Sequenz | L-Arg-L-APPA | L-Val-L-Arg-L-APPA | L-APPA |

Löslichkeit: Rhiz. A, B und APPA sind in Wasser und 35 % Ethanol leicht löslich, mässig löslich in Ethanol, höheren Alkoholen sowie unlöslich in unpolaren Lösungsmitteln.
Farbreaktion: die Verbindungen zeigen positive Ninhydrin-Reaktion und Chlor/TDM-Reaktion mit 4,4'-Tetramethyldiamino-diphenylmethan.

Dünschichtchromatographische Charakterisierung der Rhizocticine A und B und des hydrolytischen Spaltprodukte L-APPA:
Zur Dünnschichtchromatographie werden Kieselgel-Fertigeplatten 60 $F_{254}$ (Merck) verwendet.
$R_F$ -Werte werden in frisch hergestellten Fliessmittel-Systemen in mit Fliesspapier ausgelegten, gesättigten Kammern (Hersteller Firma Camag/Muttenz, Schweiz) bestimmt. Ueblicherweise wurden 2 µl einer etwa 1 proz. Lösung aufgetragen. Die Laufstrecke beträgt 8-10 cm.

Die Laufmittel und $R_F$-Werte sind in der folgenden Tabelle aufgeführt.

| Laufmittelsystem (v/v) | | |
|---|---|---|
| I) | Chlorform/Methanol/12,5 % Ammoniak | 2:2:1 |
| II) | n-Propanol/Pyridin/Eisessig/Wasser | 15:10:3:12 |
| III) | n-Butano/Pyridin/Eisessig/Wasser | 15:12:3:10 |
| IV) | n-Propanol/Wasser | 70:30 |
| V) | n-Butanol/Eisessig/Wasser | 2:1:1 |

| $R_F$ | Rhiz. A | Rhiz. B | APPA |
|---|---|---|---|
| I | 0,12 | 0,21 | 0,17 |
| II | 0,21 | 0,29 | 0,34 |
| III | 0,07 | 0,12 | 0,15 |
| IV | 0,02 | 0,04 | 0,21 |
| V | 0,10 | 0,12 | 0,16 |

Im $^1$H-und $^{13}$C-NMR-Spektrum liegen folgende chemische Verschiebungen vor:

$^1$H-NMR-Daten von Rhizocticin A (δ (ppm); $D_2O$)

| gemessene Werte | Literaturwerte* | Zuordnung |
|---|---|---|
| 4,05 | 4,13 | Arg α-H |
| 2,00 | 2,02 | Arg β-H (2H) |
| 1,80 | 1,88 | Arg γ-H |
| 3,29 | 3,27 | Arg δ-H |
| | | |
| 4,93 (1H;d;J=8,9 Hz) | 4,81 | APPA α-H |
| 5,71 (1H;m) | 5,64 | APPA β-H |
| 5,88 (1H;m) | 6,12 | APPA γ-H |
| 2,62 (2H;m;J=9,3 Hz) | 2,72 | APPA δ-H |

$^{13}$C-NMR-Daten von Rhizocticin A (δ (ppm); $D_2O$)

| gemessene Werte | Literaturwerte* | Multiplizitäten** |
|---|---|---|
| 179,8 | 175,2 | Arg (C=O) |
| 55,3 | 55,1 | Arg (CH) |
| 30,4 | 28,5 | Arg ($CH_2$)C-3 |
| 25,8 | 24,9 | Arg ($CH_2$)C-4 |
| 42,9 | 41,5 | Arg ($CH_2$)C-5 |
| 159,3 | 157,5 | Arg (C=NH) |

*B.K. Park, A. Hirota, H.Sakai, Agric. Biol. Chem. 41, 573-579 (1977)

| | | |
|---|---|---|
| 171,1 | 172,7 | APPA (C=O) |
| 55,7 (s) | 51,5 | APPA (CH) |
| 128,6 (d;J=13 Hz) | 123,5 | APPA (CH) |
| 131,4 (d;J=10 Hz) | 132,9 | APPA (CH) |
| 31,6 (d;J=128 Hz) | 29,3 | APPA ($CH_2$) |

** aus Spin-Echo-Experiment

$^{13}$C-NMR-Daten von Rhizocticin B (δ (ppm); $D_2O$)

| | | |
|---|---|---|
| 179,8 | Arg | C-1 |
| 174,3 | APPA | C-1 |
| 172,2 | Val | C-1 |
| 159,5 | Arg | C-6 |
| 130,7 | APPA | C-4 (Dublett 9,5 Hz) |
| 129,4 | APPA | C-3 (Dublett 13,2 Hz) |
| 61,3 | Val | C-2 |
| 56,2 | Arg | C-2 |
| 55,9 | APPA | C-2 |
| 43,2 | Arg | C-5 |
| 31,5 | APPA | C-5 (Dublett 127 Hz) |
| 32,8 | Arg | C-3 |
| 30,8 | Val | C-3 |
| 26,9 | Arg | C-4 |
| 20,5 | Val | C-4 |
| 19,5 | Val | C-4' |

Beispiel 7: Isolierung von Rhizocticin C und D

Bei Isolaten von Rhizocticin B nach Sephadex G-10-Reinigung liegt Mikroheterogenität vor, d.h. die N-terminale Aminosäure Valin ist dann zu 0,5-5% durch Leucin und durch Isoleucin ersetzt. Mittels HPLC lassen sich aus dem Gemisch die ebenfalls antifungischen Tripeptide Rhizocticin C (L-Ile-L-Arg-L-APPA) und Rhizocticin D (L-Leu-L-Arg-L-APPA) in geringen Mengen isolieren.

Säule: RP18 Nucleosil, 5µ; 4,6 x 250 mm, Vorsäule 4,6 x 10 mm Mobile Phase: 0,01 M $KH_2PO_4$, isokratisch; Fluss 1 ml/Min.

Zuordnung und Nachweis erfolgt durch gaschromatographische AS-Bestimmung. $R_F$-Werte (DC-System II): Rhizocticin C $R_F$ = 0,31
Rhizocticin D $R_F$ = 0,28.

Beispiel 8: Herstellung von L-2-Amino-5-phosphono-3-cis-pentensäure (L-APPA) aus den Rhizocticinen durch Hydroylse

7 mg Rhizocticin A werden in 1 ml 6N HCl 18 h bei 110°C hydrolysiert und im Stickstoffstrom bis zur Trockene abgeblasen.

Das Konzentrat wird in 200 µl Wasser aufgenommen und zur Nachreinigung (Abtrennung von Arginin) auf eine Dowex 50 WX8-Ionenaustauschersäule ($H^+$-Form, 50-100 mesh, 65 x 8 mm) aufgegeben. Während Arginin gebunden wird, lässt sich L-APPA bereits mit deionisiertem Wasser (pH 6) eluieren. Die dünnschichtchromatographisch einheitlichen Fraktionen (DC-Kontrolle auf Kieselgel, Gelbfärbung mit Ninhydrin, $R_F$ = 0,16 in System V) werden vereinigt und lyophilisiert. Es werden 3,1 mg farblose L-APPA erhalten.

Auf analoge Weise kann man die Rhizocticine B, C und D spalten und aus den Spaltproduktion L-APPA gewinnen.

$^1H$-NMR-Daten von L-APPA aus der HPLC-Trennung des Hydrolysats von Rhizocticin A (δ (ppm); $D_2O$; 400,13 MHz)

| gemessene Werte | Literaturwerte* | chem. Verschiebung |
|---|---|---|
| 2,68 (2H;m;m;J=7,5 Hz) | 2,72 (2H;d;d;J=8,4 Hz) | δCH |
| 4,59 (1H;d;J=9,8 Hz) | 4,81 (1H;d;J=9,5 Hz) | αCH |
| 5,63 (1H;m) | 5,64 (1H;d/t;J=5,2, 9,5 Hz) | βCH |
| 6,04 (1H;m) | 6,12 (1H;m) | γCH |

$^{13}C$-NMR-Daten von L-APPA aus der HPLC-Trennung des Hydrolysats von Rhizocticin A (δ (ppm); $D_2O$; 100,6 MHz)

| gemessene Werte | Literaturwerte* | |
|---|---|---|
| 176,19 (s) | 172,7 (s) | C-1 |
| 54,03 (s) | 51,5 (s) | C-2 |
| 126,48 (d;J=13 Hz) | 123,5 (d;J=12 Hz) | C-3 |
| 134,15 (d;J=10 Hz) | 132,9 (d;J=11 Hz) | C-4 |
| 31,23 (d; J=127 Hz) | 29,3 (d;J=127 Hz) | C-5 |

*B.K. Park, A. Hirota, H.Sakai, Agric. Biol. Chem. 41, 573-579 (1977)

Die Konfigurationsbestimmung erfolgte indirekt durch gaschromatographischen Vergleich des hydrierten Hydrolysegemisches enthaltend die L-5-Phosphono-valeriansäure mit D,L-5-Phosphono-valeriansäure. Gaschromatographische Bedingungen: Chirasil-Val, Temperatur Programm 5 Min 80°C isotherm, dann 3°/Min. bis 220°C; Trägergas $H_2$ (0,7 bar); (TFA/Trimethylsilyl)-Derivate.

Beispiel 9: Herstellung von L-2-Amino-5-phosphono-3-cis-pentensäure (L-APPA) aus den Rhizocticinen durch Umsetzung mit Thermolysin

7 mg Rhizocticin A in 1,5 ml 0,05N N-Ethylmorpholiniumacetat, 0,01M $CaCl_2$ x $6H_2O$ (pH 7,8) werden mit 200 µl Thermolysin (2 mg/ml) versetzt. Bei T = 41°C ist die Hydrolyse nach 30 h beendet (Ionenaustauscher-Aminosäureanalyse). Durch Zugabe verdünnter HCl bis auf pH 3 wird das Enzym desaktiviert, die Lösung am Rotationsverdampfer im Vakuum eingedampft, der Rückstand in wenig 0,05N Essigsäure aufgenommen und auf eine Sephadex G-25-Säule (10 x 250 mm, Laufm. 0,05N Essigsäure) aufgetragen. Die mit Ninhydrin auf der DC-Platte (System V) gelb anfärbbaren APPA-Fraktionen werden gesammelt und eingedampft.

Das Konzentrat wird wie in Beispiel 8 beschrieben aufgearbeitet. Es werden 3,1 mg farblose L-APPA erhalten.

Auf analoge Weise lässt sich L-APPA durch Umsetzung mit Carboxypeptidase A und Trypsin aus den Rhizocticinen gewinnen. Je nach Reaktionsbedingungen sind bis zur quantitativen Hydrolyse 3-5 Tage Inkubation erforderlich.

Reaktionsbedingungen für Umsetzungen mit Enzymen:
Enzym/Substrat-Verhältnis 5-10 %
Inkubationstemperatur T = 37°C; für Reaktion mit Carboxypeptidase A:
T = 29°C
Puffer: 0,05N N-Ethylmorpholiniumacetat, 0,01M $CaCl_2$ x $6H_2O$, pH 7,8
Substrat-Konzentration: 0,5 mg/ml

Die enzymatische Umsetzung wird in geeigneten Zeitintervallen verfolgt, indem der Reaktionslösung jeweils Aliquots (10 µl) entnommen und auf pH 2,5 mit 1N HCl zur Beendigung der Reaktion angesäuert werden. Zur Analyse werden die Proben einer automatisierten Ionenaustauscher-Aminosäureanalyse zugeführt.

Beispiel 10: Enzymatische Umwandlung von Rhizocticin B in A

Die enzymatische Hydrolyse von Rhizocticin B (L-Val-L-Arg-L-APPA) zu Rhizocticin A unter Abspaltung von Valin erfolgt mit Pronase aus Streptomyces griseus, einer Peptidase geringer Spezifität. Der enzymatische Abbau von L-Val-L-Arg-L-APPA erfolgt quantitativ innerhalb weniger Minuten (ca. 15 Min.) unter Erhalt der antifungischen Aktivität. Durch Abspaltung der N-terminalen Aminosäure Valin wird Rhizocticin A gebildet. Die Hydrolyse wird dünnschichtchromatographisch und durch Ionenaustauscher-Aminosäureanalyse bestätigt. Für das gebildete antibiotisch aktive Dipeptid ist nach 12 h noch kein Aktivitätsverlust feststellbar. Auch das native Rhizocticin A bleibt bei der Pronase-Behandlung inert.

Die Auftrennung der Spaltprodukte von Rhizocticin nach Pronase-Behandlung erfolgt auf dem Aminosäure-Analysator oder nach den zuvor beschriebenen Methoden.

Beispiel 11: Darstellung von Rhizocticin A (L-Arg-L-APPA) ausgehend von L-APPA)

Zu 51 µMol (16 mg) Boc-L-Arg-OH x HCl x $H_2O$ gibt man 51 µMol (6,9 mg) HOBt gelöst in 250 µl DMF. Unter Rühren werden 10,7 mg DCC zugegeben, 30 Min. bei RT gerührt und von unlöslichem DCH abfiltriert. Das Filtrat wird zu 40 µMol (8 mg) L-APPA, gelöst in 200 µl DMF, gegeben und nach Zugabe von 2 Aequivalenten (4 µl) N-Ethylmorpholin 4 h bei RT geführt. Das Lösungsmittel wird am Rotationsverdampfer im Hochvakuum abgedampft. Der Rückstand wird mit 2 ml Wasse digeriert und abfiltriert. Das Filtrat wird auf pH 7 eingestellt, auf eine Dowex 1X4-Säule (OH-Form, 7 x 50 mm) gegeben, mit Wasser und dann mit 0,1N Essigsäure gewaschen und das Syntheseprodukt mit 2N Essigsäure eluiert. Die Fraktionen werden am Rotationsverdampfer eingedampft und lyophilisiert. Man erhält Boc-L-Arg-L-APPA; $R_F$ = 0,28 (Kieselgel, n-Propanol/Pyridin/Eisessig/Wasser 15:10:3:12).

11 µMol (5 mg) Boc-L-Arg-L-APPA werden mit 1 ml TFE/Dichlormethan 1:1 15 Min. bei RT gerührt. Es wird am Rotationsverdampfer im Hochvakuum abgedampft, 3 ml Dichlormethan zugegeben und abgedampft. Der Rückstand wird in 0,01N HCl aufgenommen und lyophilisiert. Man erhält die Titelverbindung L-Arg-L-APPA (als Hydrochlorid); $R_F$ = 0,21 (Kieselgel, N-Propanol/Pyridin/Eisessig/Wasser 15:10:3:12).

Beispiel 12: Darstellung von Rhizocticin B (L-Val-L-Arg-L-APPA) ausgehend von L-Arg-L-APPA

20 µMol (6,2 mg) Boc-L-Val-OSu werden in 1 ml DMF gelöst, 40 µMol (4 µl) N-Ethylmorpholin und 13 µMol (5 mg) L-Arg-L-APPA x HCl zugegeben und 16 h bei RT gerührt. Die Reaktionsmischung wird am Rotationsverdampfer im Hockvakuum abgedampft, der Rückstand in 2 ml Wasser aufgenommen und dreimal mit je 2 ml Essigester extrahiert.

Die wässrige Phase wird zur Trockene eingeengt, in 0,01N HCl aufgenommen und lyophilisiert. Man erhält Boc-L-Val-L-Arg-L-APPA; $R_F$ = 0,23 (Kieselgel, n-Butanol/Eisessig/Wasser 2:1:1).

9 µMol (5 mg) Boc-L-Val-L-Arg-L-APPA werden mit 1 ml TFE/Dichlormethan 1:1 30 Min. bei RT gerührt. Die Reaktionsmischung wird am Rotationsverdampfer im Hockvakuum abgedampft. Der Rückstand wird in 200 µl DMF aufgenommen. Nach Zugabe von etherischer HCl (2 ml) fällt das farblose Produkt aus. Man erhält die Titelverbindung L-Val-L-Arg-L-APPA (als Hydrochlorid); $R_F$ = 0,12 (Kieselgel, n-Butanol/Eisessig/Wasser 2:1:1).

Beispiel 13:

Analog zu den Beispielen 11 und 12 können die folgenden Tripeptide, bzw. ihre Hydrochloride, hergestellt werden:

L-Ile-L-Arg-L-APPA
L-Leu-L-Arg-L-APPA
L-Ala-L-Arg-L-APPA
L-HomoArg-L-APPA
L-Val-L-HomoArg-L-APPA
L-Ile-L-HomoArg-L-APPA
L-Leu-L-HomoArg-L-APPA

Beispiel 14: Darstellung von L-Val-L-Lys-L-APPA ausgehend von L-APPA

Eine Lösung von 40 µMol (18,7 mg) Fmoc-L-Lys(Boc)-OH, 40 µMol (5,4 mg) HOBt und 8,3 mg DCC in 1 ml DMF wird 30 Min. bei RT gerührt, abfiltriert und das Filtrat zu 40 µMol (8 mg) L-APPA, gelöst in 0,5 ml DMF, gegeben. Nach 4 h Rühren bei RT wird am Rotationsverdampfer im Hockvakuum abgedampft, der Rückstand in 250 µl DMF aufgenommen, vom Unlöslichen abfiltriert und auf eine Sephadex LH20-Säule (200 x 8 mm, Laufmittel DMF) aufgetragen. Die chromatographisch einheitlichen Fraktionen (DC-Kontrolle auf Kieselgel in Chloroform/Methanol/Wasser 65:25:4 $R_F$ = 0,19) werden eingedampft, in 5 ml t-Butanol aufgenommen und lyophilisiert.

Eine Lösung von 18 µMol (12 mg) Fmoc-L-Lys(Boc)-L-APPA-OH in 2 ml 10 % Piperidin in DMF wird 30 Min. bei RT gerührt und am Rotationsverdampfer im Hockvakuum abgedampft. Der Rückstand wird in 0,5 ml DMF aufgenommen und mit 4 ml Diethylether und 4 ml Petrolether (30-50°) versetzt. Bei 4°C fällt das farblose L-Lys(Boc)-L-APPA aus; $R_F$ = 0,25 (Kieselgel, Chloroform/Methanol/12,5 % Ammoniak 2:2:1).

Eine Lösung von 12 µMol (5 mg) L-Lys(Boc)-L-APPA und 20 µMol (6,3 mg) Boc-L-Val-OSu in 1 ml DMF wird mit 19 µMol (2 µl) N-Ethylmorpholin versetzt und 12 h bei RT geführt. Das Reaktionsgemisch wird am Rotationsverdampfer im Hockvakuum abgedampft, der Rückstand mit 2 ml Wasser digeriert, dreimal mit je 2 ml Essigester extrahiert und die wässrige Phase lyophilisiert.

10 µMol (6,2 mg) Boc-L-Val-L-Lys(Boc)-L-APPA werden mit 1 ml TFE versetzt, nach 30 Min. wird am Rotationsverdampfer im Hockvakuum abgedampft, der Rückstand in 1 ml DMF aufgenommen und mit 3 ml Diethylether gefällt. Der Niederschlag wird 0,01N HCl aufgenommen und lyophilisiert. Man erhält L-Val-L-Lys-L-APPA (als Hydrochlorid); $R_F$ = 0,34 (Kieselgel, n-Propanol/Pyridin/Eisessig/Wasser 15:10:3:12).

Auf die gleiche Weise können die folgenden Tripeptide, bzw. ihre Hydrochloride, hergestellt werden:

L-Ile-L-Lys-L-APP
L-Leu-L-Lys-L-APP
L-Ala-L-Lys-L-APP
L-Val-L-Orn-L-APPA
L-Ile-L-Orn-L-APPA
L-Leu-L-Orn-L-APPA
L-Ala-L-Orn-L-APPA

Beispiel 15: Darstellung von L-Lys-L-APPA

Eine Lösung von 40 µMol (8 mg) L-APPA und 70 µMol (29,8 mg) Boc-L-Lys(Boc)-OSu in 2 ml DMF wird mit 153 µMol (17 µl) N-Ethylmorpholin versetzt und 12 h bei RT gerührt. Die Reaktionsmischung wird am Rotationsverdampfer im Hockvakuum abgedampft, der Rückstand in 0,5 ml DMF aufgenommen und auf eine Sephadex LH20-Säule (200 x 10 m, Laufmittel DMF) aufgetragen. Die chromatographisch einheitlichen Fraktionen (DC-Kontrolle auf Kieselgel in n-Butanol/Eisessig/Wasser 2:1:1; $R_F$ = 0,43) werden vereinigt, eingedampft, in t-Butanol aufgenommen und lyophilisiert.

Eine Mischung von 9,6 µMol (5 mg) Boc-L-Lys(Boc)-L-APPA mit 0,5 ml TFE wird 30 Min. bei RT gerührt, am Rotationsverdampfer eingedampft, in 0,01N HCl aufgenommen und lyophilisiert. Man erhält L-Lys-L-APPA (als Hydrochlorid); $R_F$ = 0,15 (Kieselgel, n-Butanol/Eisessig/Wasser 2:1:1).

In gleicher Weise lässt sich L-Orn-L-APPA, bzw. sein Hydrochlorid, darstellen.

Beisipel 16:

Trockenampullen oder Vialen enthaltend 0,5 g Rhizocticin A als Wirkstoff kann man folgendermassen herstellen:

Zusammensetzung: (für 1 Ampulle oder Viale) Wirkstoff 0,5 g
Mannit 0,5 g

Eine sterile wässrige Lösung bestehend aus Wirkstoff und Mannit wird unter aseptischen Bedingungen in 5 ml-Ampullen oder 5 ml-Vialen eingefüllt, worauf diese verschlossen und geprüft werden.

Kurze Beschreibung der Figuren

Fig. 1: Fermentation von Bacillus subtilis ATCC 6633 im 20 1-Massstab (PL-Medium, 27°C).

Aktivität gegen Paecilomyces variotii Tü 137 (mm Hemmhofdurchmesser), Aktivität gegen Saccharomyces cerevisiae Tü 125 (mm Hemmhofdurchmesser), Sediment (%), pH, $pO_2$ (%).

Fig. 2: Fermentation von Bacillus subtilis ATCC 6633 im 200 1-Massstab (PL-Medium, 27°C).

Aktivität gegen Paecilomyces variotii Tü 137 (mm Hemmhofdurchmesser), pH, Mannitgehalt (g/l).

## Ansprüche

1. Rhizocticine der Formel

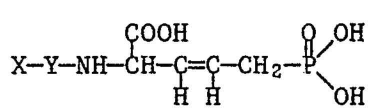

(I),

worin X Wasserstoff oder einen hydrophoben Aminosäurerest und Y einen basischen Aminosäurerest bedeuten, wobei die C2-Atome der Aminosäurereste die L-Konfiguration besitzen, sowie geschützte Derivate und Salze davon.

2. Rhizocticine der Formel I nach Anspruch 1, worin X Wasserstoff, L-Val, L-Ile, L-Leu oder L-Ala und Y L-Arg, L-Lys, L-Orn oder L-HomoArg bedeuten, sowie Salze davon.

3. Rhizocticin A der Formel I nach Anspruch 1, worin X Wasserstoff und Y L-Arg bedeuten, sowie Salze davon.

4. Rhizocticin B der Formel I nach Anspruch 1, worin X L-Val und Y L-Arg bedeuten, sowie Salze davon.

5. Rhizocticin C der Formel I nach Anspruch 1, worin X L-Ile und Y L-Arg bedeuten, sowie Salze davon.

6. Rhizocticin D der Formel I nach Anspruch 1, worin X L-Leu und Y L-Arg bedeuten, sowie Salze davon.

7. Rhizocticin der Formel I nach Anspruch 1, worin X L-Val und Y L-Lys bedeuten, sowie Salze davon.

8. Die Rhizocticine L-Ala-L-Arg-L-APPA, L-HomoArg-L-APPA, L-Val-L-HomoArg-L-APPA, L-Ile-L-HomoArg-L-APPA, L-Leu-L-HomoArg-L-APPA, L-Ile-L-Lys-L-APPA, L-Leu-L-Lys-L-APPA, L-Ala-L-Lys-L-APPA, L-Val-L-Orn-L-APPA, L-Ile-L-Orn-L-APPA, L-Leu-L-Orn-L-APPA und L-Ala-L-Orn-L-APPA, worin L-APPA den Rest der L-2-Amino-5-phosphono-3-cis-pentensäure bedeutet, oder Salze davon, nach Anspruch 1.

9. Verfahren zur Herstellung von Rhizocticinen der Formel

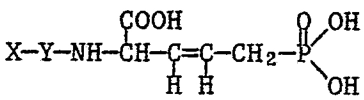 (I),

worin X Wasserstoff oder einen hydrophoben Aminosäurerest und Y einen basischen Aminosäurerest bedeuten, wobei die C2-Atome der Aminosäurereste die L-Konfiguration besitzen, sowie geschützte Derivate und Salze davon, dadurch gekennzeichnet, dass man

a) einen Rhizocticine produzierenden Bakterienstamm in einen Nährmedium, dass gegebenenfalls eine Aminosäure X oder Y oder ein Dipeptid X-Y enthält, züchtet und aus der Kulturbrühe das gewünschte Rhizocticin oder Rhizocticingemisch isoliert, oder

b) in beliebiger Reihenfolge die Aminosäuren X, Y und L-2-Amino-5-phosphono-3-cis-pentensäure, oder geschützte Derivate davon, peptidartig miteinander verknüpft, oder

c) zur Herstellung eines Rhizocticins der Formel I, worin X Wasserstoff ist, aus einem Rhizocticin der Formel I, worin X einen hydrophoben Aminosäurerest darstellt, X abspaltet,

wenn notwendig, die Schutzgruppe(n) abspaltet, und ein erhaltenes Rhizocticin der Formel I in eine Salz oder ein erhaltenes Salz in die freie Verbindung überführt.

10. Verfahren nach Anspruch 9a, dadurch gekennzeichnet, dass als Bakterienstamm Bacillus subtilis ATCC 6633 benutzt wird.

11. Verfahren nach Anspruch 9a, dadurch gekennzeichnet, dass als Nährmedium das PL-Medium benutzt wird.

12. Verfahren nach Anspruch 9a, dadurch gekennzeichnet, dass man die Rhizocticine A, B, C und/oder D isoliert.

13. Verfahren nach Anspruch 9b, dadurch gekennzeichnet, dass man L-APPA mit einer an der Aminogruppe geschützten Aminosäure H-Y-OH oder einem an der/den Aminogruppe(n) geschützten Dipeptid H-X-Y-OH, worin X ein hydrophober Aminosäurerest ist, umsetzt, und aus dem erhaltenen Di-bzw. Tripeptid die Schutzgruppe(n) abspaltet.

14. Verfahren nach Anspruch 13, dadurch gekennzeichnet, dass die BOC-oder Fmoc-Schutzgruppe und als Kondensationsmittel DCC benutzt wird.

15. Verfahren nach Anspruch 13, dadurch gekennzeichnet, dass die BOC-oder Fmoc-Schutzgruppe benutzt wird und die Carboxylgruppe in aktivierter Form als N-Hydroxysuccinimidester vorliegt.

16. Verfahren nach Anspruch 15, dadurch gekennzeichnet, dass die Schutzgruppen BOC durch Behandeln mit Trifluoressigsäure und die Schutzgruppen Fmoc durch Behandeln mit Piperidin abgespalten werden.

17. Verfahren nach Anspruch 9c, dadurch gekennzeichnet, dass der hydrophobe Aminosäurerest X mittels Pronase abgespalten wird.

18. Extrakte und Konzentrate enthaltend Rhizocticine in angereicherter Form, erhältlich aus Kulturbrühen gemäss dem Verfahren von Anspruch 9a durch Anreicherung der Rhizocticine mittels Ionenaustauschern oder Adsorberharzen und Ausschlusschromatographie.

19. Extrakte und Konzentrate gemäss Anspruch 18, enthaltend im wesentlichen die Rhizocticine A, B, C und D.

20. Verwendung der Rhizocticine oder Rhizocticine enthaltenden Extrakte oder Konzentrate nach Anspruch 1 oder 18 als Fungizide, Nematizide oder Herbizide.

21. Pharmazeutische Präparate enthaltend ein Rhizocticin nach Anspruch 1 oder ein physiologisch annehmbares Salz davon.

22. Pflanzenschutzmittel enthaltend ein Rhizocticin, nach Anspruch 1, oder einen Extrakt oder ein Konzentrat enthaltend Rhizocticin nach Anspruch 18, oder ein pflanzenverträgliches Salz davon.

23. Herbizides Mittel enthaltend ein Rhizocticin nach Anspruch 1, oder einen Extrakt oder ein Konzentrat enthaltend Rhizocticine nach Anspruch 18, oder ein Salz davon.

24. Verfahren zur Herstellung optisch reiner L-2-Amino-5-phosphono-3-cis-pentensäure (L-APPA), dadurch gekennzeichnet, dass man ein durch Fermentation eines Rhizocticine produzierenden Bakterienstammes erhaltenes Rhizocticin oder Rhizocticingemisch hydrolytisch spaltet und die L-APPA in optisch reiner Form isoliert.

25. Verfahren nach Anspruch 24, dadurch gekennzeichnet, dass man Rhizocticine, z.B. Rhizocticin A mit Thermolysin spaltet und die optisch reine L-APPA isoliert.

26. Verfahren nach Anspruch 24, dadurch gekennzeichnet, dass man Rhizocticine, z.B. Rhizocticin A mit Carboxypeptidase A oder Trypsin spaltet.

27. Optisch davon L-2-Amino-5-phosphono-3-cis-pentensäure (L-APPA) und Salze davon.

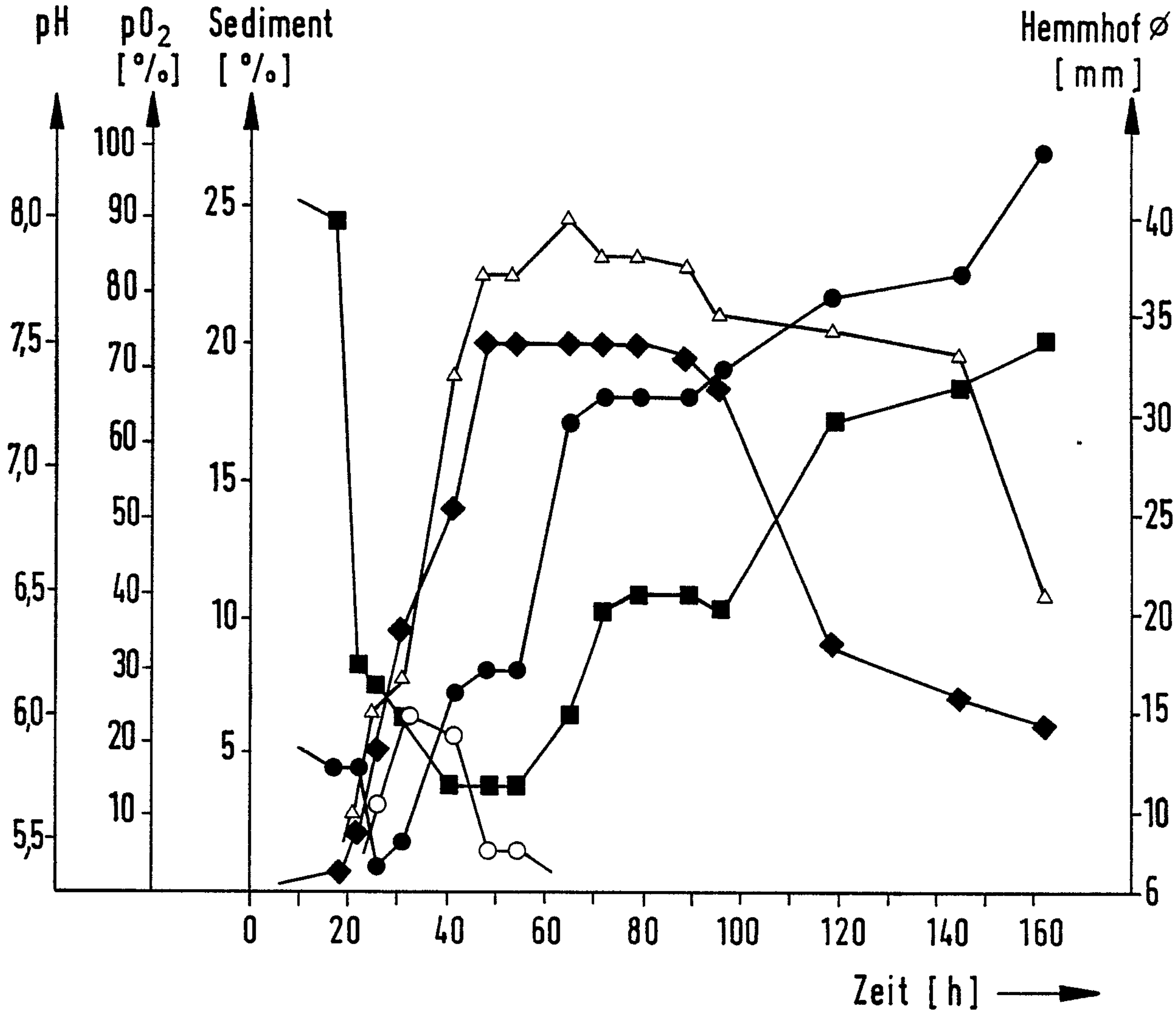


FIG.1

Fermentation von *Bacillus subtilis* ATCC 6633 im 20 l-Maßstab (PL-Medium, 27°C).
△—△ Aktivität gegen *Paecilomyces variotii* Tü 137 (mm Hemmhofdurchmesser),
○—○ Aktivität gegen *Saccharomyces cerevisiae* Tü 125 (mm Hemmhofdurchmesser),
◆—◆ Sediment (%), ●—● pH, ■—■ $pO_2$ (%).

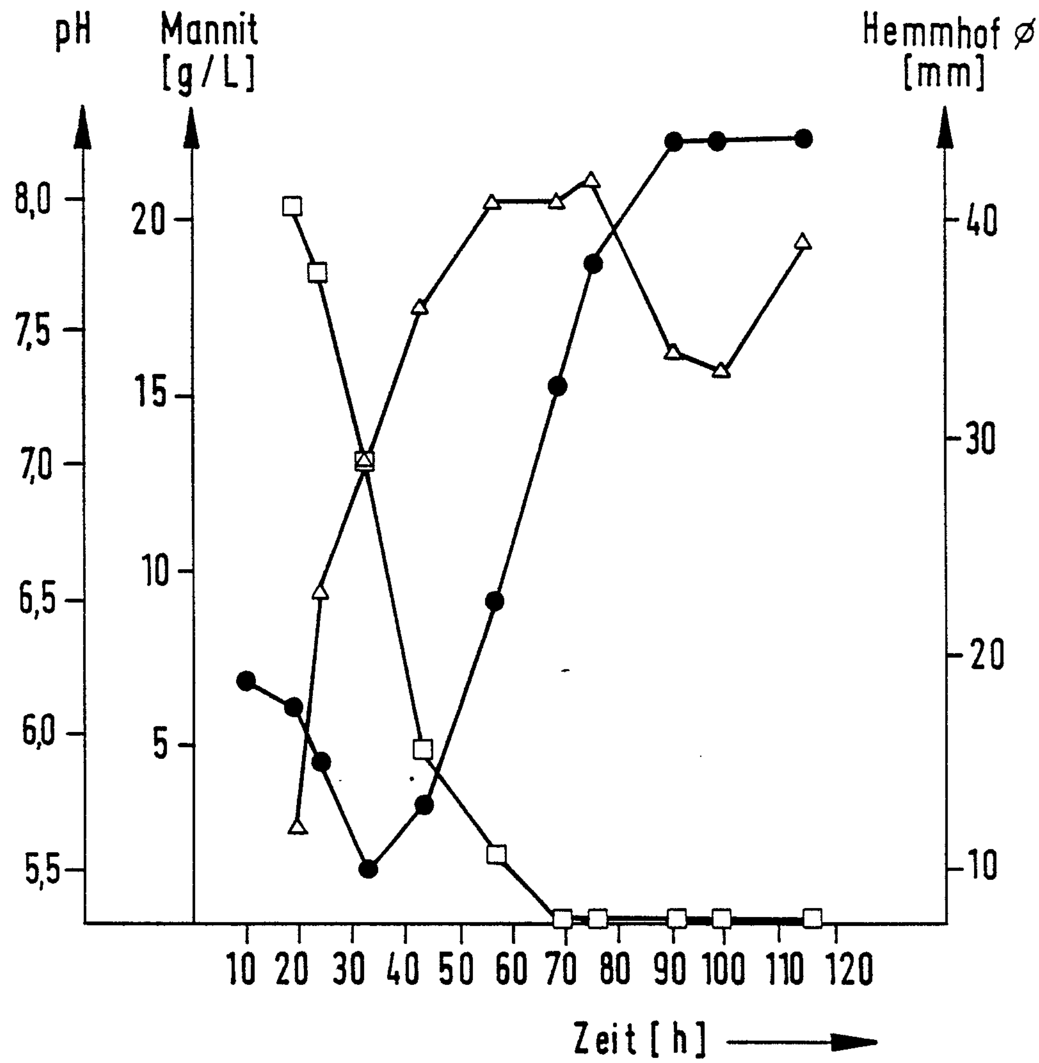


FIG. 2 Fermentation von *Bacillus subtilis* ATCC 6633 im 200 l-Maßstab (PL-Medium, 27°C).
△—△ Aktivität gegen *Paecilomyces variotii* Tü 137 (mm Hemmhofdurchmesser), ●—● pH,
□—□ Mannitgehalt (g/l).